# EUROPÄISCHE PATENTSCHRIFT

(11) **EP 1 659 968 B1**
(45) Veröffentlichungstag und Bekanntmachung des Hinweises auf die Patenterteilung: **28.03.2012**
(21) Anmeldenummer: 04764424.0
(22) Anmeldetag: 24.08.2004
(51) Int. Cl.: A61B 18/14, A61B 5/042, A61B 5/055, A61B 6/03, A61B 8/14, G01S 15/89

(54) **VORRICHTUNG ZUR VISUELLEN UNTERSTÜTZUNG EINER ELEKTROPHYSIOLOGISCHEN KATHETERANWENDUNG IM HERZEN**
DEVICE FOR VISUALLY ASSISTING THE ELECTROPHYSIOLOGICAL USE OF A CATHETER IN THE HEART
DISPOSITIF D'ASSISTANCE VISUELLE A L'UTILISATION ELECTROPHYSIOLOGIQUE D'UN CATHETER DANS LE COEUR

(30) Priorität: 01.09.2003 DE 10340546
(43) Veröffentlichungstag der Anmeldung: 31.05.2006
(73) Patentinhaber: Biosense Webster, Inc., Diamond Bar, CA 91765 (US)
(72) Erfinder: PREISS, Assaf, 42920 Bet Yizhaq (IL); RAHN, Norbert, 91301 Forchheim (DE); KARMI, Yuval, 38386 Hadera (IL); KILLMANN, Reinmar, 91301 Forchheim (DE); FUIMAONO, Kristine, 08091 West Berlin, NJ (US); HAYAM, Gal, 36501 Tivon (IL); SAUER, Frank, Princeton, New Jersey 08540 (US); XU, Chenyang, Allentown, New Jersey 08501 (US)
(74) Vertreter: Tunstall, Christopher Stephen
(86) Internationale Anmeldenummer: PCT/EP2004/009446
(87) Internationale Veröffentlichungsnummer: WO 2005/027766

(56) Entgegenhaltungen:
- EP-A- 0 945 104
- WO-A-00/25672
- DE-A- 19 621 540
- US-A1- 2003 004 405
- US-A1- 2003 018 251
- US-B1- 6 308 097
- US-B1- 6 556 695

## Beschreibung

Die vorliegende Erfindung betrifft eine Vorrichtung zur visuellen Unterstützung einer elektrophysiologischen Katheteranwendung im Herzen, bei der während der Durchführung der Katheteranwendung bereitgestellte elektroanatomische 3D-Mapping-Daten eines zu behandelnden Bereiches des Herzens visualisiert werden.

Die Behandlung von Herzrhythmus-Störungen hat sich seit der Einführung der Technik der Katheterablation mittels Hochfrequenzstrom wesentlich gewandelt. Bei dieser Technik wird unter Röntgenkontrolle ein Ablations-Katheter über Venen oder Arterien in eine der Herzkammern eingeführt und durch Hochfrequenzstrom das die Herzrhythmus-Störungen hervorrufende Gewebe verödet. Voraussetzung für eine erfolgreiche Durchführung einer Katheterablation ist die genaue Ortung der Ursache der Herzrhythmus-Störung in der Herzkammer. Diese Ortung erfolgt über eine elektrophysiologische Untersuchung, bei der elektrische Potentiale mit einem in die Herzkammer eingeführten Mapping-Katheter ortsaufgelöst erfasst werden. Aus dieser elektrophysiologischen Untersuchung, dem so genannten elektroanatomischen Mapping, werden somit 3D-Mapping-Daten erhalten, die an einem Monitor visualisiert werden können. Die Mapping-Funktion und die Ablations-Funktion sind dabei in vielen Fällen in einem Katheter vereint, so dass der Mapping-Katheter gleichzeitig auch ein Ablations-Katheter ist.

Ein bekanntes elektroanatomisches 3D-Mapping-Verfahren, wie es mit dem Carto-System der Fa. Biosense Webster Inc., USA, durchführbar ist, basiert auf elektromagnetischen Prinzipien. Unter dem Untersuchungstisch werden drei verschiedene magnetische Wechselfelder geringer Intensität aufgebaut. Mittels integrierter elektromagnetischer Sensoren an der Katheterspitze des Mapping-Katheters ist es möglich, die durch Katheterbewegungen induzierten Spannungsänderungen innerhalb des Magnetfeldes zu messen und mit Hilfe mathematischer Algorithmen zu jedem Zeitpunkt die Position des Mapping-Katheters zu errechnen. Durch punktweises Abtasten der endokardialen Kontur einer Herzkammer mit dem Mapping-Katheter bei simultaner Erfassung der elektrischen Signale entsteht eine elektroanatomische dreidimensionale Landkarte, in der die elektrischen Signale farbkodiert wiedergegeben werden.

Die für die Führung des Katheters erforderliche Orientierung des Bedieners erfolgt in der Regel bisher über fluoroskopische Visualisierung. Da die Position des Mapping-Katheters beim elektroanatomischen Mapping jederzeit bekannt ist, kann bei dieser Technik nach Erfassung einer genügend großen Anzahl von Messpunkten die Orientierung auch durch kontinuierliche Darstellung der Katheterspitze in der elektroanatomischen Landkarte erfolgen, so dass in diesem Stadium auf eine fluoroskopische Bildtechnik mit Röntgendurchleuchtung verzichtet werden kann.

Die bisher nicht optimalen Orientierungsmöglichkeiten des Bedieners bei der Führung des Katheters stellen ein grundsätzliches Problem bei der Durchführung der Katheterablation innerhalb des Herzens dar. Eine genauere Darstellung der morphologischen Umgebung während der Führung des Katheters würde einerseits die Genauigkeit bei der Katheterablation erhöhen und andererseits auch die Zeit für die Durchführung des elektroanatomischen Mapping verkürzen. Weiterhin könnte die in vielen Fällen für das elektroanatomische Mapping noch erforderliche Röntgendurchleuchtung verringert oder vermieden und damit auch die applizierte Röntgensdosis reduziert werden.

Zur Verbesserung der Orientierung des Bedieners bei der Führung des Katheters sind unterschiedliche Techniken bekannt. Bei einer Technik wird ein spezieller Katheter mit einem Ultraschall-Messkopf eingesetzt, wie er beispielsweise von der Fa. Siemens AG Medical Solutions unter der Bezeichnung Acunav angeboten wird. Über eine zweidimensionale Ultraschallerfassung der Umgebung sowie eines Teils des Katheters können Teile des zu verödenden Zielgewebes zusammen mit dem Katheter in Echtzeit visualisiert werden. Der Einsatz eines derartigen Katheters liefert allerdings keine dreidimensionale Bildinformation. Die Ultraschalldarstellung kann daher nur eingesetzt werden, um beispielsweise einen so genannten Lasso-Katheter in die Öffnung der Pulmonalvene einzusetzen. Nach der Positionierung des Lasso-Katheters kann eine Gewebever-ödung um die Öffnung einer Pulmonalvene unter Visualisierung sowohl des Lasso-Katheters als auch des Ablations-Katheters mittels Röntgenstrahlung durchgeführt werden.

Bei einer anderen bekannten Technik wird ein Lasso-Katheter ohne Unterstützung bildgebender 2D-Ultraschalltechnik an der Öffnung der Pulmonalvene platziert, indem ein Kontrastmittel über einen im linken Vorhof im Bereich der Pulmonalvenenöffnung geführten Katheter unter Röntgendurchleuchtung appliziert wird. Das Kontrastmittel verteilt sich dabei, wobei ein kleiner Teil mit dem Blutfluss über die Pulmonalvene austritt. Diese Kurzzeit-Visualisierung der Pulmonalvene ermöglicht die Platzierung des Lasso-Katheters in der Öffnung. Die Katheterablation kann nachfolgend wie bei der vorgenannten Technik durchgeführt werden.

Weiterhin ist eine Technik bekannt, bei der die Öffnung der Pulmonalvene durch elektroanatomisches Mapping des linken Vorhofs und der Pulmonalvenen lokalisiert wird, indem der Mapping-Katheter zunächst in eine Pulmonalvene eingeführt und anschließend zurückgezogen wird, bis elektrische Aktivität des Vorhofs detektiert wird. Diese Position entspricht der Position der Öffnung der Pulmonalvene, um die das Zielgewebe verödet werden soll.

Aus US 6 556 695 B1 ist eine Vorrichtung zur visuellen Unterstützung einer elektrophysiologischen Herzkatheteranwendung bekannt. Bei dieser Vorrichtung werden die Positionen der Mappingelektroden im Herzen einzeln mithilfe von im Herzen aufgenommenen Ultraschallbildern relativ niedriger Auflösung bestimmt. Die im Herzen aufgenommenen Ultraschallbilder niedriger Auflösung werden in Echtzeit mit einem zuvor aufgenommenen Bild hoher Auflösung in Registrierung gebracht, so dass die Positionen der Mappingelektroden auf dem Bild hoher Auflösung bestimmt und zusammen mit ihm angezeigt werden können. Das Vorgehen erfordert eine erhebliche Interaktion durch dem Benutzer, z. Bsp. Cursorpositionierungen auf dem Bildschirm.

Die Aufgabe der vorliegenden Erfindung besteht darin, eine Vorrichtung zur visuellen Unterstützung einer elektrophysiologischen Katheteranwendung im Herzen anzugeben, die eine verbesserte Orientierung während der Führung des Katheters bei der Katheteranwendung, insbesondere beim elektroanatomischen Mapping und/oder bei einer Katheterablation ermöglichen.

Die Aufgabe wird mit der Vorrichtung gemäß Anspruch 1 gelöst. Vorteilhafte Ausgestaltungen der Vorrichtung sind Gegenstand der Unteransprüche oder lassen sich aus der nachfolgenden Beschreibung sowie den Ausführungsbeispielen entnehmen.

Bei dem der Vorrichtung zugrunde liegendem Verfahren werden vor der Durchführung der Katheteranwendung zunächst 3D-Bilddaten einer den zu behandelnden Bereich enthaltenden Körperregion mit einem Verfahren der tomographischen 3D-Bildgebung erfasst. Aus den 3D-Bilddaten wird anschließend der zu behandelnde Bereich oder zumindest signifikante Anteile davon extrahiert. Die hierdurch erhaltenen selektierten 3D-Bilddaten und die bereitgestellten elektroanatomischen 3D-Mapping-Daten werden schließlich lage- und dimensionsrichtig zugeordnet und, vorzugsweise während der Durchführung der Katheteranwendung, gleichzeitig nebeneinander lage- und dimensionsrichtig visualisiert.

Durch die Vorrichtung wird somit dem Bediener eine Hilfestellung bei der Orientierung innerhalb des Herzens gegeben, indem die anatomischen 3D-Bilddaten und die 3D-Mapping-Daten nebeneinander in gleicher Orientierung und Skalierung an einem oder mehreren Anzeigeflächen oder Monitoren dargestellt werden. Hierdurch sind sowohl die elektrophysiologischen Eigenschaften des Gewebes als auch die zugehörige anatomische Umgebung in Echtzeit während der Katheteranwendung erkennbar. Die Visualisierung kann dabei sowohl im Kontrollraum als auch im Arbeitsraum des Herzkatheterlabors erfolgen.

Für die Erfassung der 3D-Bilddaten können beispielsweise Verfahren der Röntgen-Computer-Tomographie, der Magnetresonanz-Tomographie oder der 3D-Ultraschall-Bildgebung eingesetzt werden. Auch Kombinationen dieser Bildgebungsverfahren sind selbstverständlich möglich. Es muss lediglich darauf geachtet werden, dass die 3D-Bildaufnahmen bei der gleichen Herzphase erfolgen wie die bereitgestellten elektroanatomischen 3D-Mapping-Daten, um jeweils den gleichen Zustand des Herzens zu erfassen. Dies kann mit der bekannten Technik des EKG-Gating bei der Erfassung der Bilddaten sowie der elektroanatomischen Mapping-Daten gewährleistet werden.

Die dimensions- und lagerichtige Zuordnung der elektroanatomischen 3D-Mapping-Daten und der selektierten 3D-Bilddaten kann durch unterschiedliche Techniken erfolgen. Eine Möglichkeit besteht in der Registrierung zwischen den jeweiligen Daten durch visuelle Anpassung eines durch Segmentierung extrahierten 3D-Oberflächenverlaufs an die Darstellung der elektroanatomischen 3D-Mapping-Daten. Weiterhin lassen sich auch künstliche Marker oder natürliche markante Punkte nutzen, die in beiden Datensätzen erkennbar sind. Für die Registrierung kann neben dem zu behandelnden Bereich auch ein Nachbarbereich herangezogen werden, soweit dieser in den vorhandenen Daten enthalten ist. In einer vorteilhaften Ausgestaltung der Vorrichtung erfolgt die Registrierung in einem ersten Stadium, in dem nur ein kleinerer Teil der elektroanatomischen 3D-Mapping-Daten vorliegt, mit Hilfe von künstlichen Markern oder von markanten Punkten und in einem oder mehreren nachfolgenden Stadien, in denen bereits eine größere Anzahl elektroanatomischer 3D-Mapping-Daten vorliegt, durch Oberflächenanpassung. Auf diese Weise wird die Registrierung während der Katheteranwendung mit zunehmender Anzahl elektroanatomischer 3D-Mapping-Daten verbessert.

Die Darstellung der selektierten 3D-Bilddaten kann mittels einer Volume-Rendering-Technik erfolgen. In einer weiteren Ausgestaltung wird ein extrahierter 3D-Oberflächenverlauf durch ein Polygonnetz dargestellt, wie dies aus dem Bereich der Computergraphik bekannt ist. Die Darstellung kann mit einer einstellbaren Volume-Rendering Transferfunktion erfolgen.

Die vorliegende Vorrichtung umfasst eine oder mehrere Eingangsschnittstellen für die elektroanatomischen 3D-Mapping-Daten und die mit einem bildgebenden tomographischen Verfahren erfassten 3D-Bilddaten. Die Vorrichtung weist ein Extrahierungsmodul zur Extrahierung eines zu behandelnden Bereiches oder signifikanter Anteile davon aus den 3D-Bilddaten auf, das selektierte 3D-Bilddaten liefert. Mit diesem Extrahierungsmodul ist ein Registrierungsmodul verbunden, das für die lage- und dimensionsrichtige Zuordnung der elektroanatomischen 3D-Mapping-Daten und der selektierten 3D-Bilddaten ausgebildet ist. Mit diesem Registrierungsmodul ist wiederum ein Visualisierungsmodul verbunden, das die 3D-Mapping-Daten und die selektierten 3D-Bilddaten so zur Visualisierung bereitstellt, dass sie lage- und dimensionsrichtig nebeneinander mit einem oder mehreren Anzeigegeräten darstellbar sind.

Die einzelnen Module der Vorrichtung sind in unterschiedlichen Ausgestaltungen entsprechend zur Durchführung der nachfolgend aufgezeigten unterschiedlichen Ausführungsformen des zugrunde liegenden Verfahrens ausgebildet.

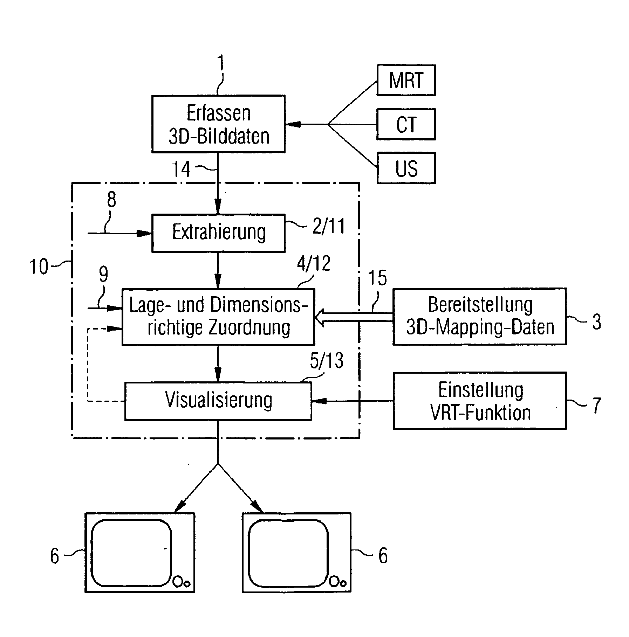

Das der Vorrichtung zugrunde liegende Verfahren sowie die Vorrichtung werden nachfolgend in Verbindung mit der Figur nochmals näher erläutert. Die Figur zeigt hierzu die einzelnen Schritte bei der Durchführung des der Vorrichtung zugrunde liegenden Verfahrens bzw. die einzelnen Module der zugehörigen Vorrichtung.

In einem ersten Schritt 1 erfolgt die Erfassung der 3D-Bilddaten der Körperregion, die insbesondere die zu behandelnde Herzkammer enthält. Bei der Erfassung dieser 3D-Bilddaten kann für eine spätere Registrierung auch ein größerer Teil des Herzens eingeschlossen werden. Die Erfassung der 3D-Bilddaten erfolgt mit einem Verfahren der tomographischen 3D-Bildgebung, wie beispielsweise Röntgen-Computer-Tomographie, Magnetresonanz-Tomographie oder 3D-Ultraschalltechniken. Bei der Erfassung der 3D-Bilddaten ist darauf zu achten, dass diese Bilddaten jeweils für die gleiche Herzphase erfasst werden, für die auch später die elektroanatomischen 3D-Mapping-Daten bereitgestellt werden. Dies wird durch EKG-Gating der Bilderfassung sowie der Erfassung der 3D-Mapping-Daten gewährleistet, beispielsweise durch Bezugnahme auf einen Prozentwert des RR-Intervalls oder auf einen festen Zeitabstand vor oder nach dem R-Peak.

Bei der Durchführung ist es wichtig, hochauflösende Bilddaten der Herzkammer zu erfassen, die während der Katheteranwendung elektroanatomisch vermessen wird. Vorzugsweise wird daher für die Erfassung der 3D-Bilddaten ein Kontrastmittel in Verbindung mit einem Testbolus oder Bolustracking eingesetzt.

Elektrophysiologische Verfahren werden in der Regel in einer der Herzkammern durchgeführt, so dass 3D-Mapping-Daten der zu behandelnden Herzkammer bereitgestellt werden. Unter den Herzkammern werden in der vorliegenden Anmeldung sowohl die Ventrikel als auch die Vorhöfe verstanden. Für die Visualisierung werden die Bilddaten dieser Herzkammer oder zumindest signifikanter Anteile davon aus den erfassten 3D-Bilddaten extrahiert. Für den Extrahierungsschritt 2 können folgende Techniken oder auch eine Kombination dieser Techniken eingesetzt werden.

In einer Ausgestaltung kann die Extrahierung 2 durch sogenanntes Volume-Clipping erfolgen. Hierbei werden interaktiv über eine Eingabeschnittstelle 8 nacheinander Einstellungen für eine Anzahl von Clip-Ebenen durchgeführt, durch die ein durch die 3D-Bilddaten erhältliches 3D-Bild auf ein Teilvolumen beschränkt wird, das die zu behandelnde Herzkammer enthält.

Eine weitere mögliche Technik zur Extrahierung 2 besteht im sogenannten Volume-Punching, bei dem nacheinander interaktiv Punching-Operationen durchgeführt werden, um irrelevante Teile des durch die 3D-Bilddaten erhältlichen 3D-Bildes auszublenden. Dies kann auch Teile des Herzens betreffen, die für die spätere Darstellung nicht relevant sind.

Eine weitere Technik besteht in der Segmentierung der 3D-Bilddaten, um einen 3D-Oberflächenverlauf der fraglichen Herzkammer sowie optional daran angrenzender Gefäße zu erhalten. Diese Segmentierung kann für eine spätere Darstellung des Oberflächenverlaufes dieser Objekte und in einer vorteilhaften Ausgestaltung auch für die lage- und dimensionsrichtige Zuordnung zu den 3D-Mapping-Daten genutzt werden.

Die Segmentierung der zu behandelnden Herzkammer - oder weiterer Kammern oder Herzgefäße - kann in Form einer 2D-Segmentierung in einzelnen Schichten erfolgen. Eine Möglichkeit besteht darin, eine vollautomatische Segmentierung aller durch das bildgebende Verfahren erhaltenen Schichten der Herzkammer durchzuführen. Alternativ hierzu können auch ein oder mehrere der Schichten interaktiv durch einen Bediener und die jeweils darauf folgenden Schichten automatisch auf Basis der Vorkenntnis der bereits segmentierten Schichten segmentiert werden. Die interaktive Segmentierung einzelner Schichten kann auch durch halbautomatische Techniken, wie beispielsweise die Technik der aktiven Konturen, unterstützt werden. Nach der Segmentierung aller Einzelschichten kann dann der 3D-Oberflächenverlauf der Herzkammer rekonstruiert werden.

Die Segmentierung kann auch als 3D-Segmentierung der zu behandelnden Herzkammer - oder weiterer Kammern oder Herzgefäße - mittels bekannter 3D-Segmentierungstechniken erfolgen. Beispiele für derartige 3D-Segmentierungstechniken sind die Schwellwerttechnik oder die Technik des Region-Growing. Falls diese vollautomatischen 3D-Segmentierungs-Algorithmen in einzelnen Fällen nicht zuverlässig arbeiten, so kann eine interaktive Eingabemöglichkeit für einen Bediener bereitgestellt werden, um beispielsweise Grauwert-Schwellwerte oder räumliche Blocker vorgeben zu können.

Die Extrahierung 2 erfolgt im Extrahierungsmodul 11 der vorliegenden Vorrichtung 10. Dieses Extrahierungsmodul 11 erhält die erfassten 3D-Bilddaten über eine entsprechende Eingangsschnittstelle 14. In gleicher Weise werden der Vorrichtung 10 über die gleiche oder eine weitere Schnittstelle 15 die 3D-Mapping-Daten in der Regel kontinuierlich während der Dauer der elektrophysiologischen Katheteranwendung zugeführt.

Die aus der Extrahierung erhaltenen selektierten 3D-Bilddaten werden dem Registrierungsmodul 12 zugeführt, in dem die lage-und dimensionsrichtige Zuordnung der selektierten 3D-Bilddaten zu den im Schritt 3 bereitgestellten 3D-Mapping-Daten erfolgt. Die 3D-Mapping-Daten werden über einen Mapping-Katheter erhalten, der über einen in die Spitze des Katheters integrierten 6D-Positionssensor 3D-Koordinaten von Oberflächenpunkten der zu behandelnden Herzkammer liefert. Derartige Katheter sind aus dem Stand der Technik für die Katheterablation bzw. das elektroanatomische Mapping bekannt. Der Katheter wird hierbei vom Bediener über Venen oder Arterien in die jeweilige Herzkammer eingeführt. Die Führung des Katheters sowie die Erfassung der 3D-Mapping-Daten ist nicht Bestandteil des vorliegenden Verfahrens. Während der Katheterablation bzw. dem elektroanatomischen Vermessen der zu behandelnden Herzkammer werden den Mapping-Daten im Laufe der Zeit zunehmend mehr Oberflächenpunkte hinzugefügt. Diese Oberflächenpunkte werden für die Rekonstruktion der morphologischen Struktur der Kammer, d.h. für dessen Visualisierung eingesetzt. Auf diese Weise entsteht im Laufe der Zeit ein zunehmend detaillierteres Bild der zu behandelnden Herzkammer aus den elektroanatomischen 3D-Mapping-Daten.

Beim Registrierungsschritt 4 im Registrierungsmodul 12 erfolgt neben der lagerichtigen Zuordnung auch eine Anpassung der Dimensionen der selektierten 3D-Bilddaten und der 3D-Mapping-Daten. Dies ist erforderlich, um eine möglichst gute Übereinstimmung der 3D-Bilddaten der Herzkammer bzw. deren Oberfläche in gleicher Orientierung, Skalierung und Form mit der entsprechenden Visualisierung der Herzkammer aus den 3D-Mapping-Daten zu erreichen. Hierfür ist in der Regel eine Transformation der selektierten 3D-Bilddaten oder der 3D-Mapping-Daten erforderlich, die drei Translationsfreiheitsgrade, drei Rotationsfreiheitsgrade, drei Skalierungsfreiheitsgrade und/oder eine Anzahl von Vektoren zur Deformation umfassen kann.

Es können künstliche Marker für die Registrierung eingesetzt werden. So können in einer Ausgestaltung vor der Erfassung der 3D-Bilddaten die künstlichen Marker an der Brust des Patienten befestigt werden. Diese Marker bleiben während der gesamten nachfolgenden Katheteranwendung an der gleichen Position fixiert. Wenigstens drei dieser Marker sind erforderlich, um eine korrekte Registrierung, d.h. Zuordnung der Bilddaten zu den Mapping-Daten zu erreichen. Hierbei müssen Marker eingesetzt werden, die sowohl in den 3D-Bilddaten erkennbar als auch durch den Positionssensor des Mapping-Systems identifizierbar sind.

Eine weitere Ausgestaltung zur Registrierung sieht vor, globale anatomische Marker, d.h. markante natürliche Punkte des zu behandelnden Bereiches oder dessen Umgebung, für eine Registrierung zu nutzen. Diese markanten Punkte müssen in den 3D-Bilddaten identifizierbar sein und werden vorzugsweise mit dem Mapping-Katheter unter Einsatz einer fluoroskopischen Bildgebungstechnik angefahren. Derartige markante Punkte sind beispielsweise die Öffnungen der Vena Cava Superior und Inferior oder des Koronarsinus. Die markanten Punkte können dann automatisch in den 3D-Bilddaten sowie den 3D-Mapping-Daten detektiert werden, so dass eine lage- und dimensionsrichtige Zuordnung dieser Daten berechnet werden kann.

Eine weitere vorteilhafte Möglichkeit zur Registrierung der 3D-Bilddaten und der 3D-Mapping-Daten besteht in der automatischen Anpassung der auf Basis dieser Daten dargestellten Oberflächen. Im Falle einer Extrahierung der zu behandelnden Herzkammer durch Segmentierung kann eine automatische Anpassung der extrahierten 3D-Oberflächenkontur der Herzkammer an die durch die 3D-Mapping-Daten erhaltene Oberflächenkontur der Herzkammer erfolgen. Bei Abweichungen in der Form der aus den 3D-Bilddaten und den 3D-Mapping-Daten erhaltenen Oberflächenkonturen können deformierende Anpassungsalgorithmen auf die Oberflächenkontur aus den 3D-Bilddaten oder auf die Oberflächenkontur aus den 3D-Mapping-Daten angewendet werden, um die gegenseitige Anpassung zu verbessern.

Die Oberflächenanpassung kann beispielsweise durch Minimierung von Punktabständen zwischen Oberflächenpunkten der Mapping-Daten und Oberflächenpunkten der aus den 3D-Bilddaten extrahierten 3D-Oberflächenkontur erfolgen (Punkt-zu-Punkt-Anpassung). Alternativ kann die Anpassung auch durch Minimierung von Punktabständen zwischen Oberflächenpunkten der Mapping-Daten und interpolierten Oberflächenpunkten der 3D-Bilddaten durchgeführt werden (Punkt-zu-Oberfläche-Anpassung).

Für die Durchführung der Oberflächenanpassung ist eine gute Oberflächenrepräsentation der zu behandelnden Herzkammer durch die 3D-Mapping-Daten erforderlich. Da diese Daten in der Regel jedoch über eine längere Zeitdauer gesammelt werden, d.h. zu Beginn der Katheterablation nur wenige elektroanatomische 3D-Mapping-Daten zur Verfügung stehen, wird vorzugsweise ein mehrstufiger Prozess der Registrierung durchgeführt. Hierbei erfolgt in einer anfänglichen ersten Stufe eine Registrierung durch Marker. Die Genauigkeit der Registrierung wird dann im Verlauf des Verfahrens durch Oberflächenanpassung in einem zweiten Schritt verbessert. Selbstverständlich lassen sich auch mit zunehmender Anzahl von Mapping-Punkten weitere Schritte der Oberflächenanpassung vornehmen, durch die gegebenenfalls eine weitere Erhöhung der Genauigkeit ermöglicht wird. Diese mehrstufige Registrierung ist von Vorteil, da die Registrierung durch Oberflächenanpassung bei entsprechend guter Oberflächenrepräsentation genauer ist als die Registrierung mittels anatomischer markanter Punkte oder künstlicher Marker, eine gute Oberflächenrepräsentation durch die Mapping-Daten jedoch erst in einem späteren Verlauf des Verfahrens erhalten wird.

In der anfänglichen ersten Stufe kann auch eine Kombination aus einer Registrierung mittels Markern und einer Registrierung mittels Oberflächenanpassung erfolgen. So kann beispielsweise eine Registrierung des linken Vorhofs durch Oberflächenanpassung einer Gefäßoberfläche z.B. der Pulmonalarterie, und zusätzlich anhand markanter anatomischer Punkte des rechten Vorhofs, z.B. des Koronarsinus oder der Öffnung der Vena Cava Inferior oder der Vena Cava Superior, erfolgen.

Nach der Registrierung zwischen den 3D-Mapping-Daten und den selektierten 3D-Bilddaten werden in Schritt 5 im Visualisierungsmodul 13 die Daten so zur Visualisierung bereitgestellt, dass sie lage- und dimensionsrichtig nebeneinander mit einem oder mehreren Anzeigegeräten 6 darstellbar sind. Mit dem gestrichelten Pfeil ist in der Figur die Möglichkeit der Verfeinerung der Registrierung bzw. Überlagerung im Verlauf der Katheterablation durch einen mehrstufigen Prozess angedeutet, wie dies bereits vorangehend erläutert wurde.

Für die Visualisierung lassen sich unterschiedliche Techniken einsetzen. So kann in einer Ausgestaltung die Visualisierung der selektierten 3D-Bilddaten durch eine Volume-Rendering-Technik erfolgen, wobei die Visualisierung durch Einstellung der Volume-Rendering-Transfer-Funktion 7 beeinflussbar ist. Da die Visualisierung der 3D-Mapping-Daten die Visualisierung der Position und Orientierung des Mapping-Katheters enthält, ist es auch möglich, den selektierten 3D-Bilddaten die Darstellung der Position und Orientierung des Mapping-Katheters zu überlagern.

In einer weiteren Ausgestaltung kann bei einer Segmentierung der 3D-Bilddaten die aus den 3D-Bilddaten extrahierte Oberfläche auch als oberflächenschattierte Darstellung oder nach einer Triangulation als Polygonnetz visualisiert werden. Auch in diesem Fall ist es möglich, die Position und Orientierung des Mapping-Katheters zusammen mit dem die Oberfläche repräsentierenden Polygonnetz darzustellen.

In einer vorteilhaften Ausführungsform der Vorrichtung werden beide Visualisierungen so miteinander verknüpft, dass sie simultan verschoben, rotiert und skaliert werden können. Zusätzlich kann ein sogenannter verlinkter Cursor eingesetzt werden, der jeweils korrespondierende Positionen in der Visualisierung der 3D-Bilddaten und in der Visualisierung der 3D-Mapping-Daten zeigt. Bei Bewegung des Cursors durch einen Benutzer in einer der Visualisierungen bewegt sich der Cursor dann entsprechend in der anderen Visualisierung.

Weiterhin kann der Mapping-Katheter, dessen Darstellung in den 3D-Mapping-Daten enthalten ist und der in der Visualisierung dieser Daten erkennbar ist, wie bereits angedeutet, bei entsprechender Registrierung zwischen den 3D-Bilddaten und den 3D-Mapping-Daten auch der Visualisierung der selektierten 3D-Bilddaten überlagert werden. Auf diese Weise kann die Position und Orientierung dieses Katheters auch jederzeit in der Visualisierung der selektierten 3D-Bilddaten erkannt werden.

## Patentansprüche

1. Vorrichtung zur visuellen Unterstützung bei einer elektrophysiologischen Katheteranwendung im Herzen mit
- einer oder mehreren Eingangsschnittstellen (14,15), welche konfiguriert sind, um elektroanatomische 3D-Mapping-Daten eines zu behandelnden Bereichs und 3D-Bilddaten zu empfangen,
- einem Extrahierungsmodul (11), das zur Extrahierung eines zu behandelnden Bereiches oder signifikanter Anteile davon aus den 3D-Bilddaten ausgebildet ist und selektierte 3D- Bilddaten liefert,
- einem mit dem Extrahierungsmodul (11) verbundenen Registrierungsmodul (12), das für eine lage- und dimensionsrichtige Zuordnung der elektroanatomischen 3D-Mapping-Daten und der selektierten 3D-Bilddaten ausgebildet ist, und
- einem mit dem Registrierungsmodul (12) verbundenen Visualisierungsmodul (13), das die 3D-Mapping-Daten und die selektierten 3D-Bilddaten so zur Visualisierung bereitstellt, dass sie lage- und dimensionsrichtig nebeneinander mit einem oder mehreren Anzeigegeräten (6) darstellbar sind,
wobei das Registrierungsmodul (12) ausgebildet ist für die automatische lage- und dimensionsrichtige Zuordnung i) anhand künstlicher Marker, die sowohl in den 3D-Bilddaten als auch in den 3D-Mapping-Daten erkennbar sind, ii) anhand markanter anatomischer Punkte , die sowohl in den 3D-Bilddaten als auch in den 3D-Mapping-Daten erkennbar sind ,iii) durch Oberflächenanpassung eines von dem Extrahierungsmodul zur Extrahierung der signifikanten Anteile des zu behandelnden Bereiches durch Segmentierung der 3D-Bilddaten erhaltenen 3D-Oberflächenverlaufes von Objekten im zu behandelnden Bereich mit einem 3D-Oberflächenverlauf aus den 3D-Mapping-Daten , oder iv) durch
einen mehrstufigen Prozess, bei dem in einem ersten Stadium die automatische lage- und dimensionsrichtige Zuordnung anhand markanter anatomischer Punkte oder künstlicher Marker erfolgt und in einem späteren zweiten Stadium durch Oberflächenanpassung eines von dem Extrahierungsmodul zur Extrahierung der signifikanten Anteile des zu behandelnden Bereiches durch Segmentierung der 3D-Bilddaten erhaltenen 3D-Oberflächenverlaufes von Objekten im zu behandelnden Bereich mit einem 3D-Oberflächenverlauf aus den 3D-Mapping-Daten verfeinert wird.

2. Vorrichtung nach Anspruch 1, **dadurch gekennzeichnet, dass** das Visualisierungsmodul (13) zur Visualisierung eines Teils eines eingesetzten Katheters innerhalb der Darstellung der selektierten 3D-Bilddaten in Echtzeit ausgebildet ist.

3. Vorrichtung nach einem der Ansprüche 1 bis 2, **dadurch gekennzeichnet, dass** das Visualisierungsmodul (13) so ausgebildet ist, dass bei einer Rotation, Verschiebung oder Skalierung einer der Visualisierungen durch einen Bediener die andere Visualisie- rungen gleichzeitig der gleichen Rotation, Verschiebung oder Skalierung unterzogen wird.

## Claims

1. Device for visual support in the case of an electrophysiology catheter application in the heart comprising
- one or more input interfaces (14, 15) which are configured for receiving electroanatomical 3D mapping data of an area to be treated and 3D image data,
- an extraction module (11) which is designed to extract an area to be treated, or significant portions of it, from the 3D image data and provides selected 3D image data,
- a registration module (12), connected to the extraction module (11), which is designed for correlation of the electroanotomical 3D mapping data and the selected 3D image data in the correct position and dimension, and
- a visualization module (13), connected to the registration module (12), which provides the 3D mapping data and the selected 3D image data for visualization such that that they can be shown in the correct position and dimension next to one another using one or more display units (6), the registration module (12) being designed for the automatic correlation in the correct position and dimension i) using artificial markers which can be identified both in the 3D image data and in the 3D mapping data, ii) using distinctive anatomical points which can be identified both in the 3D image data and in the 3D mapping data, iii) by surface matching a 3D surface profile, obtained from the extraction module for extracting the significant portions of the area to be treated by segmenting the 3D image data, of objects in the area to be treated to a 3D surface profile from the 3D mapping data, or iv) by a multi-stage process, wherein the automatic correlation in the correct position and the correct dimension is effected by means of distinct anatomatical points or artificial markers in a first stage and is refined by surface matching of a 3D surface profile, obtained from the extraction module for extracting the significant portions of the area to be treated by segmenting the 3D image data, of objects in the area to be treated to a 3D surface profile from the 3D mapping data in a later, second stage.

2. Device according to Claim 1, **characterized in that** the visualization module (13) is designed for visualizing a part of a catheter used within the representation of the selected 3D image data in real time.

3. Device according to either of Claims 1 and 2, **characterized in that** the visualization module (13) is designed so that when a user rotates, moves or scales one of the visualizations the other visualization is simultaneously subjected to the same rotation, movement or scaling.

## Revendications

1. Dispositif d'assistance visuelle à l'utilisation électrophysiologique d'un cathéter dans le coeur avec
- une ou plusieurs interfaces d'entrée (14, 15) configurées pour recevoir des données de cartographie électroanatomique 3D d'une région à traiter et des données d'images 3D,
- un module d'extraction (11) développé pour l'extraction d'une région à traiter ou une partie significative de celle-ci à partir des données d'images 3D et pour fournir des données d'images 3D sélectionnées,
- un module d'enregistrement (12) relié au module d'extraction (11), développé pour une attribution des bonnes dimension et position des données de cartographie électroanatomique 3D et des données d'images 3D sélectionnées, et
- un module de visualisation (13) relié au module d'enregistrement (12), qui prépare les données de cartographie 3D et les données d'images 3D sélectionnées pour la visualisation de manière à pouvoir les représenter avec les bonnes dimension et position côte à côte avec un ou plusieurs afficheurs (6),
le module d'enregistrement (12) étant développé pour affiner l'attribution automatique des bonnes dimension et position i) à l'aide de marqueurs artificiels reconnaissables dans les données d'images 3D de même que dans les données de cartographie 3D, ii) à l'aide de points anatomiques marquants reconnaissables dans les données d'images 3D de même que dans les données de cartographie 3D, iii) par adaptation de surface d'une évolution de surface 3D d'objets dans la région à traiter, obtenue par le module d'extraction pour l'extraction des parties significatives de la région à traiter par segmentation des données d'images 3D, avec une évolution de surface 3D provenant des données de cartographie 3D, ou iv) par un procédé en plusieurs étapes dans lequel on réalise dans une première étape l'attribution automatique à l'aide de points anatomiques marquants ou de marqueurs artificiels et dans une deuxième étape ultérieure par adaptation de surface d'une évolution de surface 3D d'objets dans la région à traiter, obtenue par le module d'extraction pour l'extraction des parties significatives de la région à traiter par segmentation des données d'images 3D, avec une évolution de surface 3D provenant des données de cartographie 3D.

2. Dispositif selon la revendication 1, **caractérisé en ce que** le module de visualisation (13) est développé pour la visualisation en temps réel d'une partie d'un cathéter mis en oeuvre à l'intérieur de la représentation des données d'images 3D sélectionnées

3. Dispositif selon une des revendications 1 à 2, **caractérisé en ce que** le module de visualisation (13) est développé de telle sorte que lors d'une rotation, d'un déplacement ou d'une mise à l'échelle d'une des visualisations par un opérateur, les autres visualisations sont simultanément soumises à la même rotation, au même déplacement ou à la même mise à l'échelle.
